Europäisches Patentamt

European Patent Office

Office européen des brevets

(19)

(11) Veröffentlichungsnummer: **0 282 994**
**A2**

(12) **EUROPÄISCHE PATENTANMELDUNG**

(21) Anmeldenummer: **88104180.0**

(22) Anmeldetag: **16.03.88**

(51) Int. Cl.⁴: **A61M 1/36**

(30) Priorität: **16.03.87 DE 3708511**

(43) Veröffentlichungstag der Anmeldung:
**21.09.88 Patentblatt 88/38**

(84) Benannte Vertragsstaaten:
**AT CH GB LI**

(71) Anmelder: **Kratzer, Michael, Dr.**
**Leopoldstrasse 56**
**D-8000 München 40(DE)**

(72) Erfinder: **Kratzer, Michael Dr.**
**Leopoldstrasse 56**
**D-8000 München 40(DE)**
Erfinder: **Unsöld, Eberhard Dr.**
**Dr. Hofmeisterstrasse 16a**
**D-8042 Oberschleissheim(DE)**

(74) Vertreter: **von Puttkamer, Nikolaus, Dipl.-Ing.**
**Pienzenauerstrasse 2**
**D-8000 München 80(DE)**

(54) **Einrichtung zur selektiven Zerstörung von Zellen.**

(57) Die Erfindung betrifft eine Einrichtung zur selektiven Zerstörung bzw. Inaktivierung von Zellen, bei der Zellen aufeinanderfolgend mit einem Lichtstrahl (61, 62) mit einer für die Zellen unschädlichen Leistung beleuchtet werden. Eine Detektoreinheit (7) weist wenigstens einen Sensor (71, 72) auf, der die an einer bestimmten Zelle aufgrund der Beleuchtung reflektierte und/oder gestreute und/oder emittierte und/oder transmittierte Strahlung (78) erfaßt und ein Detektorsignal (75, 76) erzeugt, wenn er eine vorgegebene Strahlung oder Fluoreszenz ermittelt. Bei Vorliegen des Detektorsignales (75, 76) ist ein weiterer Lichtstrahl (10) mit einer Leistung und Wellenlänge, die zur Zerstörung bzw. Inaktivierung von Zellen geeignet sind, aktivierbar und durch ein optisches System einer Abtasteinrichtung (2) auf die bestimmte Zelle projizierbar, die sich zusammen mit den Zellen in einer Abbildungsebene (21) befindet.

Fig. 2

## Einrichtung zur selektiven Zerstörung von Zellen

Die Erfindung betrifft eine Einrichtung zur selektiven Zerstörung von Zellen nach dem Oberbegriff des Patentanspruches 1.

Aus der DE-OS 53 31 969 ist ein Durchfluß-Zytometrie-Gerät zum Nachweis interessierender biologischer Teilchen in einer Probe unbekannter Teilchen bekannt, bei dem auf die Teilchen ein Lichtstrahl einer Lichtquelle gerichtet wird und durch eine Meßeinrichtung lichtbezogene Daten erfaßt werden, wenn der Lichtstrahl auf ein Teilchen trifft. Dabei erfaßt die Meßeinrichtung unter anderem die von bestimmten Teilchen emittierte Fluoreszenz.

Aus der DE-PS 33 31 017 geht ein Verfahren zur Unterscheidung verschiedenartiger Zell-Subpopulationen hervor, bei der Antikörper - Proteine mit Fluorochromen markiert werden, die markierten Anitkörper - Proteine mit einer Probe von Zellen, in denen spezifische Rezeptoren für die markierten Antikörper - Proteine vermutet werden, kombiniert werden, die Fluorochrome durch geeignete Techniken angeregt werden und die Zellen aufgrund der emittierten Fluoreszenz zur Klassifizierung analysiert werden.

Die Aufgabe der vorliegenden Erfindung besteht darin, eine Einrichtung zu schaffen, bei der Zellen insbesondere Zellen des Blutes optisch charakterisiert und selektiv mit Lichtstrahlen zerstört werden, um eine Therapie zu erzielen oder die Interaktion von Blut Zellen zu studieren.

Diese Aufgabe wird durch eine Einrichtung der Eingangs genannten Art gelöst, die durch die in dem kennzeichnenden Teil des Patentanspruches 1 angegebenen Merkmale gekennzeichnet ist.

Ein wesentlicher Vorteil der vorliegenden Erfindung besteht darin, daß es erstmals möglich wird, in Abhängigkeit von bestimmten Krankheitsbildern oder Fragestellungen bestimmte, das Krankheitsbild negativ beeinflußende oder verursachende Zellen, insbesondere Blutzellen, selektiv zu vernichten und auf diese Weise eine Besserung oder Heilung des Krankheitsgeschehens zu erreichen. Vorzugsweise kann dies nach Art einer Blutwäsche dadurch erfolgen, daß einem Patienten das Blut aus dem Körper abgezweigt und der erfindungsgemäßen Einrichtung zugeführt wird, in der die die Krankheit verursachenden oder beeinflussenden Blutzellen, bei denen es sich bspw. um bestimmte Lymphozytenpopulationen handelt, selektiv zerstört werden, und daß das Blut danach wieder in den Blutkreislauf des Patienten eingeleitet wird. Durch Zerstören dieser Blutzellen sollen die Krankheitssymptome gebessert oder es soll eine Heilung erzielt werden. Insbesondere ist es denkbar, daß mit Hilfe der erfindungsgemäßen Einrichtung erstmals auch Krankheiten wie Leukämien, Abstoßungsphänomene bei Transplantationen, AIDS und Autoimmun-Erkrankungen erfolgreich therapiert werden können

Ein wesentlicher Vorteil der erfindungsgemäßen Einrichtung besteht darin, daß Krankheitsbilder, insbesondere die genannten Krankheitsbilder therapiert werden können, ohne daß die Gesundheit des Patienten in anderer Weise durch Nebenwirkungen gefährdet wird, wie dies beispielsweise bei der Chemotherapie der Fall ist.

Vorteilhafterweise ermöglicht es die Erfindung, daß unterschiedliche Krankheitsbilder mit ein und derselben Einrichtung therapiert werden können, wobei lediglich in Abhängigkeit von der zu therapierenden Krankheit die Ausnutzung der charakteristischen Eigenschaften der einzelnen Blutzellen bzw. die Anwendung unterschiedlicher Antikörper RNA-Sonden oder sonstiger Markierungsstoffe erforderlich ist.

Die vorliegende Einrichtung kann vorteilhafterweise sowohl zur Behandlung von statistisch verweilendem Blut als auch von fließendem Blut verwendet werden. Es ist auch denkbar, mit der vorliegenden Einrichtung Zellsuspensionen in großem Maßstab zu behandeln.

Die Erfindung eignet sich vorteilhafterweise such zur Reinigung biotechnologisch einsetzbarer Zellsuspensionen. Zudem kann die vorliegende Einrichtung auch vorteilhaft auf dem Gebiete der chromosomenanalytischen Trennung von Zellen eingesetzt werden.

Ein weiterer Vorteil besteht darin, daß die erfindungsgemäße Einrichtung im wesentlichen aus auf dem Markt befindlichen Komponenten herstellbar ist.

Im folgenden werden die Erfindungen deren Ausgestaltungen im Zusammenhang mit den Figuren näher erläutert. Es zeigt:

Fig. 1 ein Blockschaltbild zur Erläuterung der Funktion der Erfindung ;

Fig. 2 ein Blockschaltbild einer bevorzugten Ausführungsform der erfindungsgemäßen Einrichtung; und

Fig. 3-6 Weiterbildungen der Erfindung.

Zusammengefaßt ist die Erfindung im wesentlichen durch die folgenden Schritte gekennzeichnet. Dabei wird das Blut beispielhaft als "Zellensuspension" zur Erläuterung herangezogen.

1) Das Blut eines Patienten wird aus dem Kreislauf, bspw. durch einen extrakorpularen Kreislauf, abgezweigt.

2) Aus dem Blut wird eine einzelne Zellgruppe, z.B. Erythrozyten, Leukozyten, Lymphozyten, oder Thrombozyten, herausgezogen, weil sie für bestimmte Krankheitsbilder relevante Zellen enthalten kann. Die restlichen Blut Zellen werden in den Blutkreislauf zurückgeführt.

3) Die herausgezogene Gruppe enthält für das Krankheitsbild relevante und nicht relevante Zellen, die morphologisch oder physikalisch nicht voneinander unterschieden werden können. Aus diesem Grunde erfolgt die Selektion der relevanten Blutzellen durch Anwendung von fluoreszierenden Antikörpern, DNA-Sonden, RNA-Sonden oder sonstigen Markierungsmitteln direkt oder indirekt.

4) Alle Blutzellen der Blutzellengruppe werden in die Brenn-bzw. Abbildungsebene eines optischen Systems gebracht und mit einem Lichtstrahl einer für die Blutzellen unschädlichen Leistung aufeinanderfolgend beleuchtet. Aufgrund ihrer Markierung emittieren die relevanten Blutzellen eine Fluoreszenz.

5) Das von den markierten Blutzellen emittierte Licht wird von einem Sensor erfaßt.

6) Durch Einstrahlen eines Lichtstrahles mit einer höheren Leistung, die zur Zerstörung von Blutzellen ausreicht, in das optische System, können markierte bzw. relevante Zellen, die für das Krankheitsbild verantwortlich sind, vernichtet werden. Dabei wird der Lichtstrahl mit der höheren Leistung dadurch aktiviert, daß die Fluoreszenz der markierten bzw. relevanten Zellen nach deren Anregung durch den Lichtstrahl mit der niedrigen Leistung zur Erzeugung eines Aktivierungssignales detektiert wird.

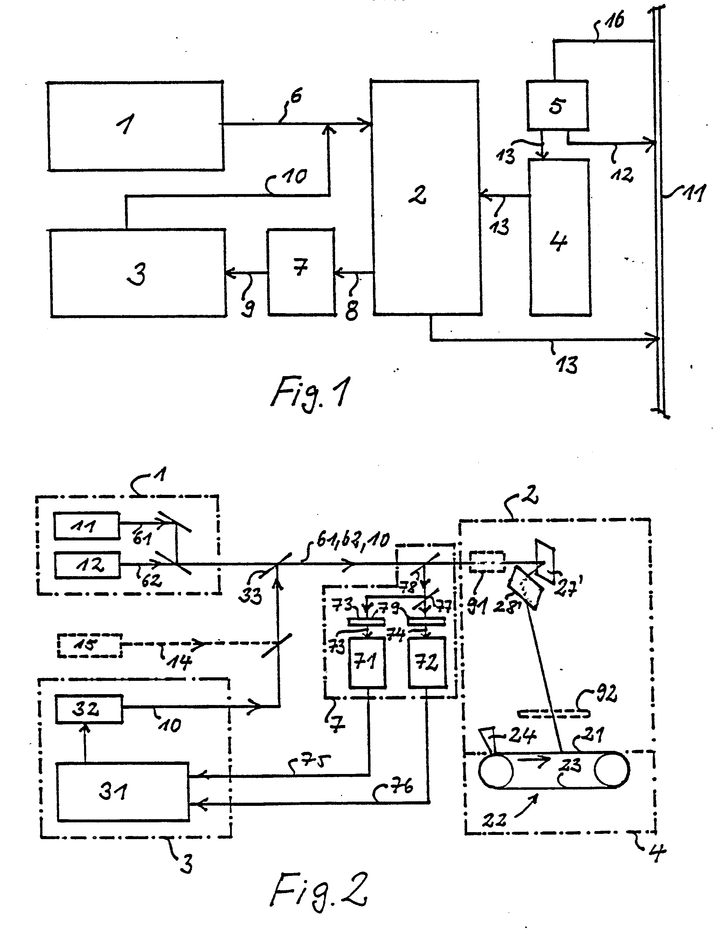

Im folgenden wird nun die Funktion der Erfindung zunächst im Zusammenhang mit dem Blockschaltbild der Fig. 1 näher erläutert. In der aus der Fig. 1 ersichtlichen Weise besteht die erfindungsgemäße Einrichtung im wesentlichen aus einem Analyseteil 1, einer Abtasteinrichtung 2, einem aktiven Teil 3, einer Zellzufuhreinrichtung 4 und einer Detektoreinheit 7. Dabei kann der Zellzufuhreinrichtung 4 zweckmäßigerweise ein Zellselektor 5 vorgeschaltet sein.

Der Analyseteil 1 erzeugt wenigstens einen Lichtstrahl 6 mit einer für Zellen, insbesondere fur Blutzellen, unschädlichen Leistung. Dieser Lichtstrahl 6 wird durch die Abtasteinrichtung 2 auf die Abbildungsebene eines optischen Systems projiziert und dort vorzugsweise in den zwei Richtungen X und Y eines karthesischen Koordinatensystems gescannt, wenn die Abbildungsebene selbst unbeweglich ist, oder nur in einer Richtung des genannten Koordinatensystems gescannt, während die Abbildungsebene in der anderen Richtung bewegt wird. Es ist auch denkbar, daß der Lichtstrahl 6 durch die Abtasteinrichtung 2 nur auf die Abbildungsebene projiziert wird und daß nur die Abbildungsebene in einer vorgegebenen Richtung bewegt wird so daß vom Lichtstrahl 6 keine Fläche, wie dies bei den beiden vorhergehenden Fällen zutrifft, sondern nur eine Linie erfaßt wird.

Die Detektoreinheit 7 erfaßt die von den einzelnen, durch den Lichtstrahl 6 in der Abbildungsebene aufeinanderfolgend angeregten Zellen emittierte Fluoreszenz und/oder die an diesen Zellen reflektierte Lichtstrahlung und/oder die an diesen Zellen gestreute Strahlung und/oder die von den Zellen transmittierte Strahlung. In der Fig. 1 sind die genannte Fluoreszenz und die genannten Strahlen mit 8 bezeichnet. Die Detektoreinheit 7 erzeugt wenigstens ein Detektorsignal 9; das der erfaßten Emission und/oder einer bestimmten gestreuten oder reflektierten Strahlung 8 entspricht.

Das wenigstens eine Detektorsignal 9 wird an Steuereinrichtungen des aktiven Teiles 3 angelegt, wobei ein Aktivierungssignal erzeugt wird, das eine in dem aktiven Teil 3 enthaltene Lichtquelle aktiviert, die einen Lichtstrahl 10 erzeugt, dessen Leistung so groß ist, daß sie zur Zerstörung oder Inaktivierung von Zellen ausreicht. Dieser Lichtstrahl 10 wird vorzugsweise dem optischen System der Abtasteinrichtung 2 zugeführt, so daß er durch dieses System ebenfalls auf denselben Punkt der Abbildungsebene projiziert wird, wie der Lichtstrahl 6. Dies bedeutet, daß bei Anregung einer Zelle durch den Lichtstrahl 6 und bei Empfang einer durch diese Zelle emittierten, vorgegebenen Fluoreszenz oder einer an dieser Zelle reflektierten oder gestreuten vorbestimmten Strahlung das genannte Aktivierungssignal und der Lichtstrahl 10 erzeugt werden, weil es sich bei der Zelle um eine für ein Krankheitsbild verantwortliche, markierte relevante Zelle handelt. Diese wird dann durch die Leistung des eingeschalteten Lichtstrahles 10 zerstört. Es ist auch denkbar, die Schritte der Analyse (Projektion des Strahles 6) und die Schritte des aktiven Eingriffes (Strahl 10) räumlich und zeitlich getrennt durchzuführen.

Die Zellzufuhreinrichtung 4 führt die zu untersuchenden oder zu therapierenden Zellen der Abbildungsebene der Abtasteinrichtung 2 zu. Dabei können die zugeführten Zellen, wenn es sich um Blutzellen handelt, in der schematisch dargestellten Weise dem Blutkreislauf 11 eines Patienten entnommen werden (Weg 16), und nach der durchgeführten Therapie oder Untersuchung diesem Blutkreislauf wieder zugeführt werden. Es kann dabei der dargestellte Vorselektor 5 Verwendung finden. Dieser zieht aus dem ihm zugeführten Blut, beispielsweise durch eine Zentrifugenoperation, eine einzelne Zellgruppe heraus, die für das zu therapierende Krankheitsbild relevante Zellen enthalten kann. In diesem Fall werden nur die entnommenen, relevanten Blutzellen durch die Zellzufuhreinrichtung in die Abbildungsebene der Abtasteinrichtung gebracht. Die restlichen Blutzellen des aus dem Blutkreislauf abgezweigten Blutes werden diesem wieder direkt hinter dem Vorselektor 5 zugeführt (Weg 12), während die therapierten Blutzellen von der Abbildungsebene wieder in den Blutkreislauf eingebracht werden (Weg 13). Durch die Vorselektion wird erreicht, daß auf der Abbildungsebene nicht alle Blutzellen des Blutes, sondern nur die Blutzellen einer einzelnen Zellgruppe, in der bevorzugt die für das zu therapierende Krankheitsbild verantwortlichen Zellen enthalten sind, abgetastet werden müssen. Dadurch kann der gesamte Abtastvorgang wesentlich beschleunigt werden.

Im Zusammenhang mit der Fig. 2 werden nun bevorzugte Ausführungsformen der vorliegenden Erfindung näher erläutert. Einzelheiten der Fig. 2, die bereits im Zusammenhang mit der Fig. 1 erläutert wurden, sind in der entsprechenden Weise bezeichnet.

In der Analyseneinheit 1 wird als Lichtquelle 11 besonders bevorzugt eine Laserquelle verwendet, da in diesem Fall ein besonders scharfer und kleiner Brennfleck in der Abbildungsebene 21 der Abtasteinrichtung 2 erzeugbar ist. Dies führt beispielsweise zur Erzeugung eindeutiger Fluoreszenzen an den aufeinanderfolgend in der Abbildungsebene 21 abgetasteten Zellen.

Besonders gut eignet sich als Laserquelle ein Argonlaser, dessen Anregungsfrequenz beispielsweise 488 nm entspricht. Mit Fluoresceinisothiocyanat markierte Antikörper emittieren dann, wenn sie mit der 488 nm entsprechenden Anregungsfrequenz angeregt werden, eine Fluoreszenz bei 550 nm.

Mit Laserquellen sind ohne weiteres Brennflecken erzeugbar, deren Durchmesser etwa 10 µm oder weniger betragen kann.

An der Stelle einer einzigen Lichtquelle 11 können mehrere Lichtquellen, z.B. zwei Lichtquellen 11, 12 vorgesehen werden, die Lichtstrahlen 61, 62 erzeugen, deren Leistung für die Zellen unschädlich sind. Bei der Verwendung zweier Lichtquellen 11, 12 können die Zellen in der Abbildungsebene gleichzeitig mit unterschiedlichen Frequenzen angeregt werden, so daß beispielsweise zwei Fluoreszenzen mit unterschiedlichen Wellenlängen erzeugt werden. Dies kann für spezielle Fragestellungen bzw. Therapien vorteilhaft sein. Außerdem kann dies für die Erzeugung besonders störungsfreier Detektorsignale 9 von Bedeutung sein, weil z.B. durch Subtraktion der durch entsprechende Sensoren der Detektoreinheit 7 erfaßten Signale, bei denen es sich beispielsweise um Fluoreszenzsignale handelt, Untergrundsignale eliminiert werden können.

Die beiden Lichtstrahlen 61, 62 werden beispielsweise zu einem Strahl zusammengeführt und gemeinsam vom optischen System der Abtasteinrichtung 2 aufgenommen. Das optische System der Abtasteinrichtung 2 projiziert einen Brennfleck der Lichtquelle 11 oder der Lichtquellen 11, 12 auf die Abbildungsebene 21. Die Abtasteinrichtung 2 weist beispielsweise zwei an sich bekannte Ablenkspiegel 27', 28' auf, von denen einer durch eine Antriebsvorrichtung für die Ablenkrichtung in der X-Richtung und der andere durch eine weitere Antriebseinrichtung für die Ablenkung in der Y-Richtung in Schwingungen versetzt wird. An der Stelle der Ablenkspiel 27', 28' können auch sogenannte elektro-optische Deflektoren vorgesehen werden, die ebenfalls an sich bekannt sind. Des weiteren kann die Ablenkung in der X-und Y-Richtung in an sich bekannter Weise auch durch rotierende Hologramme realisiert werden.

Durch Vorsehung nur eines Ablenkspiegels oder nur eines elektro-optischen Deflektors kann die Auslenkung des Lichtstrahles 61 bzw. der Lichtstrahlen 61, 62 in nur einer Richtung (beispielsweise in der X-Richtung) erfolgen, wenn in diesem Falle die Möglichkeit gegeben ist, daß die Abbildungsebene 21 selbst in der anderen Richtung (beispielsweise der Y-Richtung) bewegt wird. Dies kann zweckmäßigerweise durch einen - schematisch dargestellten Bandförderer 22 erfolgen, wobei die Abbildungsebene in diesem Falle einem Teil der Oberfläche des Bandes 23 dieses Bandförderers 22 entspricht und die Auslenkung des Lichtstrahles 61 bzw. der Lichtstrahlen 61, 62 vorzugsweise senkrecht zur Bewegungsrichtung des Bandes 23 erfolgt. Bei der Vorsehung eines durchsichtigen Bandes 23 kann die Sensoreinrichtung 71 der später noch näher erläuterten Detektoreinheit 7 auf der der Abbildungsebene 21 gegenüberliegenden Seite des Bandes 23 vorgesehen sein. Das Blut wird im Falle der Verwendung eines Bandförderers 22 aus einer geeigneten Vorratsbehältereinrichtung 24 auf die Oberfläche des Bandes 23 zweckmäßigerweise zusammen mit dem die Blutzellen am Leben erhaltenden Nährmedium, in der Bewegungsrichtung des Bandes 23 gesehen vor dem Ort aufgebracht, an dem der bzw. die Lichtstrahlen 61, 62 gescannt werden.

Im Zusammenhang mit der Fig. 3 wird nun eine Bandfördereinrichtung beschrieben, bei der das Blut an einem Ende des Bandes 23 aufgebracht wird und bei der die Blutzellen danach während der Fortbewegung des Bandes 23 zum Abtastort 25 derart sedimentieren, daß sie gerade am Abtastort 25 in der Abbildungsebene, d.h. also auf der Bandoberfläche angeordnet sind. Um dies zu erreichen, wird die Bewegungsgeschwindigkeit des Bandes 23 in Abhängigkeit von dem Sedimentierungsvorgang eingestellt. Um ein seitliches Abfließen des Blutes vom Band 23 zu verhindern, weist dieses entsprechende Seitenwände 26 auf, so daß die Bandoberfläche den Boden eines gebildeten, etwa U-förmigen Kanales entspricht.

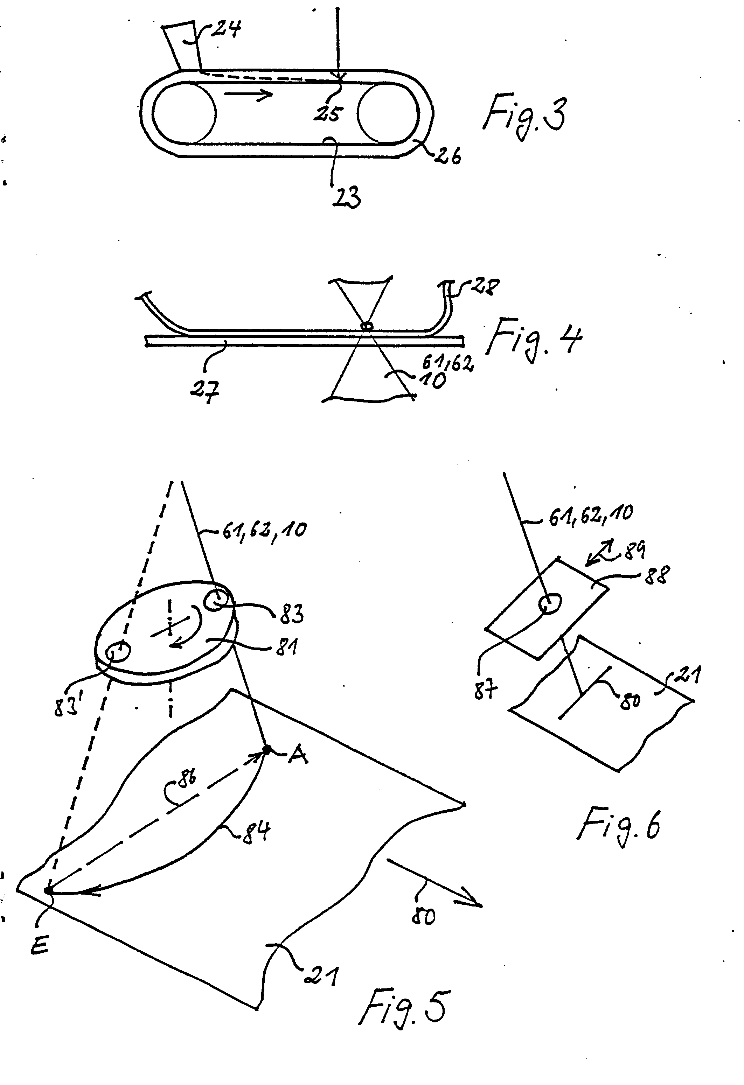

Bei einer bevorzugten Weiterbildung der Erfindung wird die Abbildungsebene 21 durch die Oberfläche einer durchsichtigen Platte 27 gebildet (Fig. 4), wobei die Abtasteinrichtung so ausgestaltet ist, daß der bzw. die Lichtstrahlen 61, 62 von unten her auf die Platte 27 auftreffen. Auf der gegenuberliegenden Oberfläche der Platte 27 kann dann das in einem durchsichtigen Kunststoffbeutel oder dergl. enthaltene Blut bzw. können die in dem genannten Beutel 28 enthaltenene, durch den Vorselektor 5 selektierten Blutzellen, aufgelegt werden. Dabei werden dann der Lichtstrahl bzw. die Lichtstrahlen 61, 62 auf die Ebene der auf den Boden des Beutels 28 oder dergl. aufliegenden Zellen fokussiert. Dabei ist es von Vorteil, daß der auf die einzelnen Zellen fokussierte Lichtstrahl 10 mit der hohen Leistung, die zur Zerstörung von relevanten Zellen führt, in dem Bereich, in dem er durch die Wand des Beutels 28 tritt, noch nicht fokussiert ist, so daß die Leistungsdichte so gering ist, daß der Beutel nicht zerstört bzw. beschädigt wird.

Der bzw. die Lichtstrahlen 61, 62 können auch auf einen feststehenden Punkt der Abbildungsebene 21 fokussiert werden, wobei das Blut bzw. die Blutzellen durch den Bereich des Punktes strömen, wie dies an sich im Zusammenhang mit sogenannten Flowkammern bekannt ist.

Die Detektoreinheit 7 weist vorzugsweise entsprechend der Anzahl der Lichtquellen 61, 62 Sensoren 71, 72 auf, die die von den Zellen emittierte Fluoreszenz oder die an den Zellen reflektierte oder gestreute Strahlung 73, 74 empfangen und beim Empfang einer bestimmten Fluoreszenz oder bestimmter Strahlungen ein Detektorsignal 75, 76 erzeugen. Vorzugsweise wird die von den Zellen emittierte Fluoreszenz oder die an den Zellen reflektierte Strahlung über das optische System der Abtasteinrichtung 2 zurückgeführt, wobei die Fluoreszenzsignale oder die reflektierten Lichtsignale 73, 74 zweckmäßigerweise durch Strahlteiler 77, 78 aus dem Strahlengang des optischen Systems zu den Sensoren 71, 72 ausgekoppelt werden. Es ist jedoch auch denkbar, die Sensoren 71, 72 bzw. die Detektoreinheit 7 seitlich von der Abbildungsebene 21 vorzusehen. Dies ist insbesondere im Falle der Auswertung der an den Zellen gestreuten Strahlung zweckmäßig.

Zur Führung der Lichtstrahlen können in an sich bekannter Weise auch Glasfasern verwendet werden.

Das Detektorsignal 75 bzw. die Detektorsignale 75, 76 werden einer Steuereinrichtung 31 des aktiven Teiles 3 zugeführt, bei der es sich beispielsweise um einen Zentralprozessor (CPU-Einheit) handelt, der aus den Detektorsignalen das Aktivierungssignal erzeugt.

Als Sensoren 71, 72 dienen vorzugsweise Photomultiplier, denen jeweils ein Filter 79 vorgeschaltet ist, das nur eine Wellenlänge, die beispielsweise genau der emittierten Fluoreszenz entspricht, durchläßt.

Das Aktivierungssignal wird der Lichtquelle 32 des aktiven Teiles 3 zugeführt, die den Lichtstrahl 10 mit der Leistung und Wellenlänge, die zur Zerstörung bzw. Inaktivierung von Zellen geeignet sind, erzeugt. Vorzugsweise handelt es sich bei dieser Lichtquelle 32 ebenfalls um eine Laserquelle, insbesondere um einen Argonlaser. Es ist auch denkbar, durch diese Lichtquelle 32 UV-Licht zu erzeugen. Das Licht 10 der Laserquelle 32 wird vorzugsweise über einen Strahlteiler 33 in den Strahlengang des optischen Systemes der Abtasteinrichtung 2 eingekoppelt.

An der Stelle der Lichtquelle 11 mit der niedrigen Leistung und der Lichtquelle 32 mit der hohen Leistung kann auch eine einzige Lichtquelle 15 vorgesehen werden, die normalerweise einen Lichtstrahl 14 mit der niedrigen Leistung erzeugt, die für die Zellen unschädlich ist, wobei die Leistung dieses Lichtstrahles 14 bei Vorliegen des Aktivierungssignales von der CPU-Einheit 31 kurzzeitig derartig vergrößerbar ist, daß sie zur Zerstörung bzw. Inaktivierung von Zellen ausreicht. Nach einer vorgegebenen Zeitperiode, die beispielsweise etwa 30 bis 40 nsec. beträgt, wird die Energie des Laserstrahles 14 jeweils wieder auf den niedrigen Energiepegel abgesenkt und es wird der Abtastvorgang fortgeführt.

Beispielsweise wird als Laserquelle 14 eine bekannte Laserquelle vom sogenannten Acousto-Optic-Cavity-Dumping-Typ verwendet, die bei Vorliegen des Aktivierungssignales bei einer Anstiegszeit von 7 nsec. einen Impuls einer hohen Energie von 36 nsec. erzeugen kann.

Im folgenden werden Lösungsmöglichkeiten erläutert, durch die erreicht wird, daß auch bei großen Auslenkungen des Brennflecks der Licht- bzw. Laserstrahlen 61, 62, 10 in der Abbildungsebene 21 eine Korrektur der spärischen Aberration ereicht wird.

Ein neuartiger Gedanke zur Lösung dieses Problems besteht in der Verwendung eines auf einer Scheibe rotierenden Objektivs, das mit der Bewegung der Ablenkspiegel 27', 28' bzw. mit der Ansteuerung der elektro-optischen Deflektoren synchronisiert wird. Fig. 5 zeigt eine derartige Anordnung. Die das Objektiv exzentrisch tragende Scheibe 81 wird so gedreht und die Ablenkspiegel 27', 28' bzw. die elektro-optischen Deflektoren werden so angetrieben bzw. angesteuert, daß stets sichergestellt ist, daß die durch das optische System der Abtasteinrichtung 2 geführten Lichtstrahlen 61, 62, 10 immer genau während einer vorgegebenen Zeitperiode der Drehung des Objektives 83 folgen. Da diese eine Kreisbahn beschreibt, ist eine Auslenkung in der X-Y-Richtung erforderlich. In der Abbildungsebene 21 entsteht dann eine kreisförmige Ablenklinie 84 des Brennfleckes bzw. der Brennflecken. Eine Abtastung in der anderen Richtung (Y-Richtung) wird dadurch erreicht, daß die Abbildungsebene 21 in der Richtung des Pfeiles 80 in Bezug auf die Ablenklinie 84 verschoben wird. Dies erfolgt beispielsweise mit dem bereits erwähnten Bandförderer 22.

Besonders bevorzugt ist dabei eine Ausführungsform, bei der auf einem Durchmesser der rotierenden Scheibe 81 vom Mittelpunkt gleichmäßig beabstandet zwei Objektive 83, 83' angeordnet sind, so daß bei einer geeigneten Synchronisierung der Scheibe 81 und der Ablenkspiegel 27', 28' bzw. der elektro-optischen Deflektoren immer gerade ein Objektiv 83 oder 83' in den in die Ausgangsposition A zurückgekehrten Strahl 61, 62, 10 gedreht wird, um dem Strahl bis zur Endposition E zu folgen. Die unterbrochene Linie 86 zeigt die Strahlrückführung von der Endposition E zur Ausgangsposition A an.

Es ist auch denkbar, an der Stelle der rotierenden Scheibe 81 mit dem Objektiv 83 bzw. den Objektiven 83 und 83' ein Objektiv 87 auf einer Schwingplatte 88 vorzusehen, die in der Richtung des Pfeiles 89 geradlinig hin-und herbewegt wird (Fig. 6). In diesem Fall ist nur ein Ablenkspiegel bzw. ein elektro-optischer Deflektor erforderlich, der mit der Hin-und Herbewegung der Platte 88 so synchronisiert wird, daß der Lichtstrahl immer durch das Objektiv 87 hindurchtritt. In der Abbildungsebene 21 entsteht dann die gerade Ablenklinie 80. Der Vorteil dieser sich drehenden Scheibe besteht darin, daß Objektive hoher Apertur verwendet werden können und somit eine hohe Empfindlichkeit erreicht werden kann.

Es ist außerdem denkbar, zur Korrektur der sphärischen Aberration in dem Strahlengang eine an sich bekannte dynamische Linse 91 anzuordnen, die in der Strahlrichtung in Abhängigkeit von einem Signal, das anzeigt, daß der Brennfleck nicht in der Abbildungsebene 21 liegt, verschiebbar ist. Auf diese Weise kann erreicht werden, daß die dynamische Linse 91 automatisch so nachgeführt wird, daß sich der Brennfleck immer genau in der Abbildungsebene 21 befindet. Derartige dynamische Linsen sind beispielsweise im Zusammenhang mit CD-Plattenspielern bekannt. Die Vorsehung einer derartigen Linse geht aus der Fig. 2 hervor.

An der Stelle der rotierenden Scheibe 81 kann im Strahlengang vor der Abbildungsebene 21 auch eine sogenannte Flachfeldlinse (F-$\theta$ -Linse) vorgesehen werden, deren Charakteristiken sich in der X-Auslenkrichtung und/oder Y-Auslenkrichtung derart ändern, daß immer sichergestellt wird, daß sich der ausgelenkte Brennfleck in der Abbildungsebene 21 befindet.

In der Fig. 2 ist eine derartige Flachfeldlinse -

schematisch dargestellt und mit 92 bezeichnet.

An der Stelle der beschriebenen Laserquelle 10 können auch Stoßwellen erzeugende Quellen Verwendung finden. Außerdem können bestimmte Zellen mit photolabilen Substanzen markiert werden, die Gifte intrazellulär freisetzen, wenn Licht geeigneter Wellenlänge und Energie als Strahl 10 auf diese auftreffen.

Bei der Erfassung und Auswertung des an den Blutzellen reflektierten oder gestreuten Lichtes kann ein Bild oder können spezielle optisch erkennbare Merkmale registriert werden und können diese mit einem zuvor gespeicherten Bild bzw. zuvor gespeicherten optischen Merkmalen, das bzw. die bestimmte zu zerstörende Blutzellen kennzeichnen, verglichen werden. Bei Übereinstimmung der ermittelten und gespeicherten Daten kann die Zerstörung der gerade vorliegenden Blutzellen durch Erzeugung des Aktivierungssignales ausgelöst werden.

Im folgenden wird nun beispielhaft eine denkbare Therapie von AIDS beschrieben:

Von HTLV-III-Viren befallene T4-Lymphozyten bauen (nach Aktivierung mit Interlencin I und Antigen) Virus-Proteine in die T4-Zellmembran ein. Daher erscheint es denkbar, die infizierten Zellen durch fluoreszenzmarkierte Antikörper gegen die Virusproteine selektiv zu markieren.

Es wird dabei beispielsweise davon ausgegangen, daß 5 l Blut, das pro $\mu$l 8000 Leukozyten enthält, therapiert wird. Dies bedeutet, daß sich in den 5 l Blut $5 \times 10^6 \times 8000 = 4 \times 10^{10}$ Leukozyten befinden. Es wird weiter angenommen, daß davon 50 %, d.h. also $2 \times 10^{10}$ Lymphozyten und davon wiederum 50 %. d.h. also $1 \times 10^{10}$ T-Lymphozyten sind. Die Blutzellen haben einen Durchmesser von etwa 10 $\mu$m. Dies bedeutet, daß der Brennfleck des zur selektiven Zellzerstörung verwendeten Laserstrahles 10 einen Durchmesser von etwa 10 $\mu$m haben soll. Derartige Brennfleckgrößen sind ohne weiteres realisierbar.

Wenn man davon ausgeht, daß $10^{10}$ Zellen dicht gedrängt nebeneinander liegen, wird eine Fläche von etwa $10^{10} \times 10 \times 10^{-6} \times 10 \times 10^{-6} = 1m^2$ bedeckt. Es soll jede Zelle selektiv innerhalb von 10.000 sec., d.h. ungefähr 2,7 Stunden, beleuchtet werden. Dies entspricht einer Frequenz von $10^{10}$ Zellen/$10^4$ gleich $10^6$ Hertz. Für die Abtastung einer Blutzelle stehen also 1 $\mu$s zur Verfügung, was technisch möglich ist.

Während der Abtastung können die Zellen durch eine nicht dargestellte Temperiereinrichtung auf beispielsweise 4°C gekühlt werden. Dadurch wird z.B. die Fluoreszenz stabilisiert.

## Ansprüche

1. Einrichtung zur selektiven Zerstörung bzw. Inaktivierung von Zellen, bei der Zellen aufeinanderfolgend mit einem Lichtstrahl (61, 62) mit einer für die Zellen unschädlichen Leistung, der von wenigstens einer Lichtquelle (11, 12) erzeugt wird, beleuchtet werden, wobei die von bestimmten Zellen aufgrund der Beleuchtung abgegebenen Strahlung durch eine Detektoreinheit (7) erfaßt wird, dadurch gekennzeichnet, daß die Detektoreinheit (7) wenigtens einen Sensor (71, 72) aufweist, der die an einer bestimmten Zelle reflektierte und/oder gestreute und/oder emittierte und/oder transmittierte Strahlung (78) erfaßt und ein Detektorsignal (75, 76) erzeugt, wenn er eine vorgegebene Strahlung oder Fluoreszenz ermittelt, daß bei Vorliegen des Detektorsignales (75, 76) eine weitere Lichtquelle (32) einschaltbar ist, deren weiterer Lichtstrahl (10) eine Leistung und Wellenlänge besitzt, die zur Zerstörung bzw. Inaktivierung von Zellen geeignet sind, und daß der weitere Lichtstrahl durch ein optisches System einer Abtasteinrichtung (2) auf eine Abbildungsebene (21) projizierbar ist, in der sich die Zellen befinden.

2. Einrichtung nach Anspruch 1, dadurch gekennzeichnet, daß als Lichtquelle (11, 12) eine Laserquelle vorgesehen ist.

3. Einrichtung nach Anspruch 1, dadurch gekennzeichnet, daß als weitere Lichtquelle eine Laserquelle vorgesehen ist.

4. Einrichtung nach Anspruch 1, dadurch gekennzeichnet, daß die Lichtquelle und die weitere Lichtquelle durch eine einzige Lichtquelle (15) gebildet sind, deren Lichtstrahl (14) von einer niedrigen, für Zellen unschädlichen Leistung, auf eine höhere, für Zellen schädliche Leistung, umschaltbar ist, wenn das Detektorsignal (75, 76) erzeugt wird.

5. Einrichtung nach Anspruch 4, dadurch gekennzeichnet, daß die einzige Lichtquelle (15) eine Laser quelle ist.

6. Einrichtung nach einem der Ansprüche 2 bis 5, dadurch gekennzeichnet, daß als Laserquellen Argon-Laserquellen vorgesehen sind.

7. Einrichtung nach einem der Ansprüche 1 bis 6, dadurch gekennzeichnet, daß das optische System der Abtasteinrichtung (2) den Lichtstrahl (61, 62) und den weiteren Lichtstrahl (10) oder den Lichtstrahl (14) der gemeinsamen Lichtquelle (15) in den Richtungen (X, Y) eines karthesischen Koordinatensystemes ablenkende Ablenkspiegel (27', 28') oder elektro-optische oder holographische Deflektoren aufweist.

8. Einrichtung nach einem der Anspruche 1 bis 6, dadurch gekennzeichnet, daß das optische System der Abtasteinrichtung (2) einen den Lichtstrahl (61, 62) und den weiteren Lichtstrahl (10) oder den Lichtstrahl (14) der gemeinsamen Lichtquelle (15) in einer ersten Richtung ablenkenden Ablenkspiegel oder elektro-optischen oder holographischen Deflektor aufweist und daß die Abbildungsebene (21) in einer zweiten Richtung bewegbar ist.

9. Einrichtung nach Anspruch 7 oder 8, dadurch gekennzeichnet, daß das optische System der Abtasteinrichtung (2) vor dem Ablenkspiegel bzw. den Ablenkspiegeln (27', 28') oder vor dem Deflektor bzw. den Deflektoren eine dynamische Linse (91) zur Korrektur der sphärischen Aberration aufweist.

10. Einrichtung nach Anspruch 7 oder 8, dadurch gekennzeichnet, daß das optische System der Abtasteinrichtung (2) zur Korrektur der sphärischen Aberration zwischen der Abbildungsebene (21) und dem Ablenkspiegel bzw. den Ablenkspiegeln oder dem Deflektor bzw. den Deflektoren eine Flachfeldlinse (92) aufweist.

11. Einrichtung nach einem der Ansprüche 1 bis 6, dadurch gekennzeichnet, daß das optische System der Abtasteinrichtung (2) den Lichtstrahl auf einen Punkt der Abbildungsebene (21) projiziert und daß die Abbildungsebene (21) in einer Richtung bewegbar ist.

12. Einrichtung nach einem der Anspruche 1 bis 11, dadurch gekennzeichnet, daß die Abbildungsebene (21) einer inneren Bodenfläche eines Gefäßes (28) entspricht, auf der die Zellen angeordnet sind, und daß die Bodenfläche auf einer stationären Platte (27) anordenbar ist oder daß die Platte in das Gefäß integriert ist.

13. Einrichtung nach Anspruch 12, dadurch gekennzeichnet, daß als Gefäß (28) ein Kunststoffbeutel oder dergl. vorgesehen ist.

14. Einrichtung nach Anspruch 12 oder 13, dadurch gekennzeichnet, daß wenigstens die Bodenfläche des Gefäßes (28) und die Platte (27) aus einem durchsichtigen Material bestehen und daß der Lichtstrahl (61, 62) und der weitere Lichtstrahl (10) oder der Lichtstrahl (14) der einzigen Lichtquelle (15) von der der Bodenfläche des Gefäßes (28) gegenüberliegenden Seite der Platte (27) her einstrahlbar sind.

15. Einrichtung nach einem der Ansprüche 1 bis 10, dadurch gekennzeichnet, daß die Abbildungsebene (21) durch wenigstens einen Teilbereich der Oberfläche eines Bandes (23) eines Bandförderers (22) gebildet ist.

16. Einrichtung nach Anspruch 15, dadurch gekennzeichnet, daß an dem Bandförderer (22) eine die Zellen auf die Oberfläche des Bandes (23) zuführende Vorratsbehältereinrichtung (24) vorgesehen ist.

17. Einrichtung nach Anspruch 15 oder 16, dadurch gekennzeichnet, daß das Band (23) aus einem durchsichtigen Material besteht und daß die Detektoreinheit (7) auf der Seite des Bandes (23) angeordnet ist, die der Abbildungsebene (21) gegenüberliegt.

18. Einrichtung nach einem der Ansprüche 15 bis 17, dadurch gekennzeichnet, daß das Band (23) an seinen Seiten nach außen vorstehende Seitenwände (26) aufweist, so daß ein Kanal gebildet wird, in den die Zellen aus der Vorratsbehältereinrichtung (24) eingebracht werden, daß die Zellen in dem Kanal zwischen der Vorratsbehältereinrichtung (24) und dem davon beabstandeten Ort (25), an dem der Lichtstrahl (61, 62), der weitere Lichtstrahl (10) oder der Lichtstrahl (14) der einzigen Lichtquelle (15) auf die Oberfläche des Bandes (23) auftrifft bei geeigneter Wahl der Vorschubgeschwindigkeit des Bandes (23) derart sedimentieren, daß sich die Zellen am Ort (25) in der Abbildungsebene (21) befinden.

19. Einrichtung nach einem der Ansprüche 1 bis 18, dadurch gekennzeichnet, daß die Detektoreinheit (7) wenigstens einen Sensor (71, 72) aufweist, der eine bestimmte an einer Zelle reflektierte oder gestreute oder transmittierte Strahlung oder eine bestimmte von einer Zelle emittierte Fluoreszenz sowie gegebenenfalls deren Polarisationsgrad zur Erzeugung eines Detektorsignales (75, 76) erfaßt.

20. Einrichtung nach Anspruch 19, dadurch gekennzeichnet, daß die dem Sensor (71, 72) zuzuführende Strahlung oder Fluoreszenz (78) aus dem Strahl des optischen Systems der Abtasteinrichtung (2) ausgekoppelt wird.

21. Einrichtung nach Anspruch 19 oder 20, dadurch gekennzeichnet, daß als Sensor (71, 72) ein Photomultiplier mit wenigstens einem vorgeschalteten Filter (79) vorgesehen ist, das nur die bestimmte Strahlung oder die bestimmte Fluoreszenz durchläßt.

22. Einrichtung nach einem der Ansprüche 1 bis 21, dadurch gekennzeichnet, daß der aktive Teil (3) einen Zentralprozessor (31) aufweist, der aus dem Detektorsignal (75, 76) ein die weitere Lichtquelle (32) kurzzeitig einschaltendes oder ein die einzige Lichtquelle (15) von der niedrigen Leistung auf die höhere Leistung umschaltendes Aktivierungssignal (34) erzeugt.

23. Einrichtung nach einem der Ansprüche 1 bis 22, dadurch gekennzeichnet, daß der Abbildungsebene (21) dem Blutkreis (11) eines Patienten entnommene Blutzellen durch die Zellzufuhreinrichtung (4) zugeführt werden.

24. Einrichtung nach Anspruch 23, dadurch gekennzeichnet, daß der Zellzufuhreinrichtung (4) ein Vorselektor (5) vorgeschaltet ist, der die Blutzellen aussondert und der Zellzufuhreinrichtung (4) zuführt, die einer Zellgruppe angehören, in der Zellen, die bestimmte Krankheitsbilder verursachen, bevorzugt enthalten sind, und daß die Zellen der Zellgruppe hinter der Abbildungsebene (21) (Weg 13) und die Zellen, die nicht der Zellgruppe angehören, hinter dem Vorselektor (5) (Weg 12) dem Blutkreislauf (11) wieder zugeführt werden.

25. Einrichtung nach einem der Ansprüche 1 bis 24, dadurch gekennzeichnet, daß eine Selektion der relevanten Zellen, die für ein bestimmtes Krankheitsbild verantwortlich sein können, durch Anwendung von fluoreszierenden Antikörpern, DNA-Sonden, RNA-Sonden oder sonstigen Markierungsmitteln erfolgt.

26. Einrichtung nach einem der Ansprüche 11 bis 25, dadurch gekennzeichnet, daß die Zellen durch die Zellzufuhreinrichtung (4) mit dem erforderlichen Medium zugeführt werden.

27. Einrichtung nach einem der Ansprüche 1 bis 26, dadurch gekennzeichnet, daß das optische System vor der Abbildungsebene (21) wenigstens ein rotierbares Objektiv (83) aufweist, das auf einer rotierbaren Scheibe (81) exzentrisch befestigt ist, und daß der eine (61, 62) und der weitere Lichtstrahl (10) oder der Lichtstrahl (14) der gemeinsamen Lichtquelle (15) durch Synchronisierung der Schwingungen des ersten und zweiten Ablenkspiegels (27', 28') bzw. der Ansteuerung der Deflektoren und der Drehzahl der Scheibe (81) während der Drehung derselben immer auf das Objektiv (83) auftreffen.

28. Einrichtung nach Anspruch 27, dadurch gekennzeichnet, daß die Scheibe (81) zwei Objektive (83, 83') aufweist, die auf einem Durchmesser der Scheibe (81) von der Drehachse derselben gleichmäßig beabstandet sind, und daß der eine (61,62) und der weitere Lichtstrahl (10) und der Lichtstrahl (14) der gemeinsamen Lichtquelle (15) den Objektiven (83, 83') aufeinanderfolgend während einer halben Umdrehung der Scheibe folgen.

29. Einrichtung nach einem der Ansprüche 1 bis 26, dadurch gekennnzeichnet, daß das optische System vor der Abbildungsebene (21) eine Schwingplatte (88) mit einem Objektiv (87) aufweist, daß die Schwingplatte (88) in einer Richtung (89) in Schwingungen versetzbar ist, und daß der eine (61,62), und der weitere Lichtstrahl (10) und der Lichtstrahl der gemeinsamen Lichtquelle (15) durch Synchronisation der Schwingung der Schwingplatte (88) und der Schwingung des einen Ablenkspiegels oder der Ansteuerung des einen Deflektors immer auf das Objektiv (87) der Schwingplatte (88) auftreffen.

30. Einrichtung nach einem der Ansprüche 1 bis 29, dadurch gekennzeichnet, daß das optische System Glasfasern zur Lichtstrahlführung aufweist.

31. Einrichtung nach einem der Ansprüche 1 bis 30, dadurch gekennzeichnet, daß der eine Lichtstrahl (61, 62) und der weitere Lichtstrahl (10) zeitlich und räumlich getrennt Zellen detektieren und zerstören bzw. inaktivieren.

Fig. 1

Fig. 2

Fig.3

Fig.4

Fig.5

Fig.6